# EUROPÄISCHE PATENTSCHRIFT

(11) **EP 0 924 301 B1**
(45) Veröffentlichungstag und Bekanntmachung des Hinweises auf die Patenterteilung: **02.03.2005**
(21) Anmeldenummer: 98123197.0
(22) Anmeldetag: 05.12.1998
(51) Int. Cl.: C12P 17/04, C07D 307/62

(54) **Enzymatische Herstellung von regioselektiven Fettsäureestern der Ascorbinsäure**
Enzymatic preparation of regioselective fatty acid esters of ascorbic acid
Préparation enzymatique d'esters régiosélectifs d'acide gras de l'acide ascorbique

(30) Priorität: 20.12.1997 DE 19757103
(43) Veröffentlichungstag der Anmeldung: 23.06.1999
(73) Patentinhaber: Goldschmidt AG, 45127 Essen (DE)
(72) Erfinder: Grüning, Burghard, Dr., 45134 Essen (DE); Hills, Geoffrey, 45355 Essen (DE)

(56) Entgegenhaltungen:
- EP-A- 0 401 704
- EP-A- 0 514 694
- EP-A- 0 551 638
- US-A- 4 705 869
- US-A- 5 455 174
- CHEMICAL ABSTRACTS, vol. 117, no. 23, 7. Dezember 1992 Columbus, Ohio, US; abstract no. 232236, NUMAZAWA, RYOZO ET AL: "Manufacture of ascorbate or erythorbate esters with esterase" XP002094893 & JP 04 141093 A (MITSUBISHI RAYON CO., LTD., JAPAN)
- CHEMICAL ABSTRACTS, vol. 69, no. 17, 21. Oktober 1968 Columbus, Ohio, US; abstract no. 66921, TANAKA, HIROYOSHI ET AL: "Acyl-substituted ascorbic acids" XP002094894 & JP 42 026633 A (SHIONOGI AND CO., LTD.)
- CHEMICAL ABSTRACTS, vol. 80, no. 7, 18. Februar 1974 Columbus, Ohio, US; abstract no. 36050, TAKAHASHI, SEIICHI: "Stable dietary oil" XP002094895 & JP 48 068602 A (TAIYO YUSHI CO., LTD.)

## Beschreibung

Gegenstand der Erfindung ist ein Verfahren zur enzymatischen Herstellung von regioselektiven Fettsäureestern der Ascorbinsäure und ausgewählte Fettsäureascorbylester.

Die Herstellung von Ascorbylfettsäureestern auf chemischem Wege gelingt nur unter extremen Bedingungen, zum Beispiel in Schwefelsäure oder in Fluorwasserstoff, siehe beispielsweise DE 27 43 526 A1, DE 33 08 922 A1 oder DE 28 54 353 C3. Derartige Prozesse sind in der technischen Durchführung bedenklich. Die Produkte bedürfen einer aufwendigen Reinigung. Produkte, die nach diesen Verfahren hergestellt werden, weisen auch nach üblichen Aufarbeitungen einen vom Verbraucher nicht akzeptierten Geruch auf.

Die EP 0 401 704 B1 beschreibt ein Verfahren zur Herstellung eines organischen Esters von Ascorbinsäure umfassend die Reaktion von Ascorbinsäure in Gegenwart von 100 bis 10000 ppm Wasser und einer organischen Säure oder einem Ester davon in einem organischen Lösungsmittel in der Gegenwart einer Esterhydrolase. Die hier berichteten Ausbeuten in den Tabellen 1 bis 4 überschreiten jedoch nicht den Wert von 2 %, so daß dieses Verfahren keinen Eingang in die Technik gefunden hat.

C. Humeau et al., Biotechnology Letters, Vol. 17, Nr. 10, 1995, S. 1091 bis 1094 beschreiben die Synthese von Ascorbylpalmitat in einem nichtwässrigen Medium mit einer immobilisierten Jipase, erhalten aus Candida antartica, als Biokatalysator. Die enzymatische Synthese wird als perfekt regioselektiv beschrieben. Es wird angegeben, daß bei Verwendung von Palmitinsäure-Methylester als Acyldonor 68 % der Ascorbinsäure umgewandelt wird. Diese Werte wurden erhalten bei einem Anfangsstoffmengenverhältnis von Ascorbinsäure zu Acyldonor von 1 mol zu 5 mol und einer sehr geringen Einsatzkonzentration. Nacharbeitungen des entsprechenden Versuchs ergaben jedoch weit geringere Reaktionsausbeuten, so daß auch dieses Verfahren nicht für eine technische Herstellung der Fettsäureester von Ascorbinsäure geeignet ist.

In den Tagungsunterlagen des 1. Workshops "Biokonversion nachwachsender Rohstoffe", 30. Juni/1. Juli 1997 Detmold, S. 57 und 58 wurden von B. Haase et al. enzymatische Synthesen von Zuckerestern vorgestellt. Es wird dargestellt, daß es in zunehmenden Maße gelingt, durch selektive, enzym-(lipase) katalysierte Veresterungen zu besser definierten, ja regioisomerenreinen Produkten zu gelangen. Als Beispiel für Zuckerester wird eine 6-O-Acyl-L-Ascorbinsäure ohne Angabe eines konkreten Herstellungsverfahrens genannt.

Die US 4 705 869 beschreibt ein Verfahren zur Herstellung von Fettsäureestern der Ascorbinsäure unter Verwendung von konzentrierter (>96%) Schwefelsäure.

In der EP 0 551 638 werden stabile flüssige Präparate fettlöslicher Substanzen beschrieben, in denen Ester der Ascorbinsäure mit langkettigen gesättigten Fettsäuren als Emulgatoren verwendet werden. Ein vergleichbares Verfahren beschreibt die JP 73-68602.

In der japanischen Patentanmeldung 67-26633 wird z.B. 5-Benzoylascorbinsäure in einer Mischung aus Pyridin und Dimethylcarbonat unter Eiskühlung mit Alkylchloriden zu den gemischten Benzoyl/Alkyl- oder Alkyl/Alkylascorbinsäureestern umgesetzt.

Die EP 0 514 694 (US 5 455 174) beschreiben ein enzymatisches Verfahren bei dem unter Normaldruck Ascorbinsäure mit einem "organic acid enol ester": R¹COO-(R²)C=CH₂ umgesetzt wird. Die Verwendung von Fettsäureenolestern soll Rückreaktionen unterbinden.

JP-A-4-141093; Mitsubishi Rayon Co. Ltd. beschreibt die Veresterung von Fettsäuren mit Ascorbinsäure unter Einsatz eines enzymatischen Katalysators in einem Lösungsmittel, das ein Azeotrop mit Wasser bildet. Die Ausbeute beträgt lediglich 37 % nach 72 h Reaktionszeit bzw. 18 % nach gleicher Reaktionsdauer, wenn das Reaktionswasser nicht während der Reaktion entfernt wird. Zudem erfordert das Verfahren die Kontrolle des Wassergehaltes in der Reaktionsmischung in einem Konzentrationsbereich von 200 bis 2000 ppm.

Demgegenüber besteht die Aufgabe der vorliegenden Erfindung darin, ein verbessertes Verfahren zur Herstellung von Ascorbylfettsäureestern mit erhöhten Ausbeuten und insbesondere im Hinblick auf technisch relevante Maßstäbe zur Verfügung zu stellen.

Die vorgenannte Aufgabe wird in einer ersten Ausführungsform der Erfindung gelöst durch ein Verfahren zur enzymatischen Herstellung von regioselektiven Fettsäureestern der Ascorbinsäure, wobei man Fettsäurealkylester in einem Lösungs- oder Suspendiermittel bei gegenüber der Atmosphäre vermindertem Druck bei Temperaturen oberhalb des Siedepunktes des entstehenden Reaktionsalkohols bei den Reaktionsbedingungen mit Ascorbinsäure umestert.

Das erfindungsgemäße Verfahren ermöglicht die Herstellung von neuen und bekannten Ascorbylfettsäureestern mit gegenüber dem Stand der Technik erhöhten Ausbeuten und in größeren Mengen. In einer Reaktionsmischung aus Ascorbinsäure und einem Fettsäurealkylester wird in einem Reaktionssystem mit einem geeigneten Lösungs- oder Suspendiermittel in Gegenwart von einem Enzym, welches die Veresterungs- bzw. Umesterungsreaktion katalysiert, Vakuum angelegt, um den freigesetzten Alkohol abzudestillieren. Dies hat die Wirkung, daß das Reaktionsgleichgewicht auf die Produktseite verschoben wird, so daß sich insgesamt die Ausbeute des Produkts gegenüber dem in JP-A-4-141093 beschriebenen Stand der Technik deutlich erhöht. Mit Hilfe der vorliegenden Erfindung sind überraschenderweise Ausbeuten im Bereich bis über 80 % möglich. Es wurde gefunden, daß die Entfernung des Reaktionsalkohols eine Verbesserung des Verfahrens bewirkt, die die aus dem Massenwirkungsgesetz heraus prinzipiell ableitbare Verbesserung deutlich übertrifft. Mögliche Ursache könnte die Vermeidung der Bildung von stark sauren wasserhaltigen Ascorbinsäurelösungen sein. Im Gegensatz zu dem in JP-A-4-141093 erfordert das erfindungsgemäße Verfahren keine Kontrolle des Wassergehaltes, obwohl durch die Anwendung eines verminderten Drucks Wasser ggf. zusammen mit anderen Reaktionsprodukten entfernt werden würde.

Wie oben angegeben, ist in der Literatur bekannt, daß Lipasen die Synthese von 6-Ascorbylpalmitat regiospezifisch katalysieren können. Die Produktivität des berichteten Reaktionssystems ist aber nicht ausreichend für eine Produktion im technischen Maßstab wegen a) der niedrigen Konzentrationen und b) der zu niedrigen Ausbeuten. Im erfindungsgemäßen Verfahren ist es möglich höhere Konzentrationen einzusetzen und nahezu vollständige Umsetzungen eines der Reaktanden, nämlich des im Unterschuß befindlichen, zu erreichen.

Die enzymatische Reaktion wird im Vergleich zu chemischen Prozessen unter milden Bedingungen durchgeführt und daher können Produkte mit erheblich besserer Qualität (geruchsfrei) hergestellt werden. Das erfindungsgemäße Verfahren läßt sich insbesondere gut zur Herstellung von ungesättigten Fettsäureestern nutzen, die von Ölsäure oder Linolsäure abgeleitet sind.

Besonders bevorzugt im Sinne der vorliegenden Erfindung und für eine Anwendung als Antioxidationsmittel in Lebensmitteln und in der Kosmetik besonders gut geeignet, sind regioselektive Fettsäureester der Ascorbinsäure, die sich von Fettsäurealkylestern ableiten, die ausgewählt sind aus geradkettigen oder verzweigten, einfach oder mehrfach ungesättigten, substituierten oder unsubstituierten Fettsäureresten mit 8 bis 24 Kohlenstoffatomen. Besonders bevorzugt im Sinne der vorliegenden Erfindung sind solche Ascorbinsäureester, deren Fettsäurereste 12 bis 18 C-Atome aufweisen. Die Alkylreste der Fettsäurealkylester weisen vorzugsweise 1 bis 3 C-Atome im Alkylrest auf. Besonders bevorzugt in diesem Sinne ist Methanol. Mit Hilfe der vorliegenden Erfindung lassen sich besonders gut ungesättigte, substituierte Fettsäureascorbylester, beispielsweise hydroxy-substituierte Ester, wie die Ricinolester herstellen.

Als Ascorbinsäure werden vorzugsweise die L-Ascorbinsäure oder die D-Ascorbinsäure eingesetzt, insbesondere die L-Ascorbinsäure.

Das Stoffmengenverhältnis von Ascorbinsäure zu Fettsäurealkylester läßt sich in einem weiten Bereich variieren. So besteht eine besonders bevorzugte Ausführungsform der vorliegenden Erfindung darin, daß man das Stoffmengenverhältnis von Ascorbinsäure zu Fettsäurealkylester im Bereich von 10 mol zu 1 mol bis 1 mol zu 10 mol einstellt. Besonders bevorzugt in diesem Sinne wird das Stoffmengenverhältnis im Bereich von 1 mol zu 1 mol bis 1 mol zu 5 mol eingestellt.

Die gemäß der vorliegenden Erfindung einzusetzenden Enzyme sind im Stand der Technik bekannt und im Handel erhältlich. Beispielsweise beschreibt die WO 90/09451 auf S. 8 im zweiten Absatz entsprechende Enzyme, die auch unter der Bezeichnung Novozym®435 oder Chirazyme L-2 erhältlich sind. In der genannten WO 90/09451 werden Enzyme, insbesondere immobilisierte Enzyme beschrieben, die ausgewählt sind aus Lipasen, Esterasen oder Proteasen. Besonders bevorzugt im Sinne der vorliegenden Erfindung sind Lipasen mit definierter Enzymkatalyse-Reaktivität für Esterbindungen, insbesondere Hydrolyse, Synthese und/oder Austausch von Esterbindungen. Das Produkt Novozym®435 der Novo Nordisk stellt ein immobilisiertes thermostabiles Lipasesystem dar, das im Handel erhältlich ist und besonders bevorzugt im Sinne der vorliegenden Erfindung eingesetzt wird.

Die Art des Lösungs- oder Suspendiermittels kann im Sinne der vorliegenden Erfindung in weiten Grenzen variiert werden. Gegebenenfalls kann auch ein flüssiger Reaktand, wie ein Fettsäurealkylester als Lösungs- oder Suspendiermittel insbesondere für die Ascorbinsäure dienen, sofern sich das gewünschte Produkt in dem System ausreichend löst. Besonders bevorzugt im Sinne der vorliegenden Erfindung werden Lösungs- oder Suspendiermittel eingesetzt, deren Siedepunkte oberhalb, insbesondere wenigstens 10 °C oberhalb des Siedepunkts des entstehenden Reaktionsalkohols bei den Reaktionsbedins mgen liegen. Wenn im Sinne der vorliegenden Erfindung die destillative Entfernung des Reaktionsalkohols angesprochen ist, so sind hierunter alle gängigen Verfahren zu verstehen, die dem Durchschnittsfachmann mit chemischen Grundkenntnissen geläufig sind. Beispielsweise ist hier die azeotrope Entfernung des Reaktionsalkohols mit geeigneten Schleppern, die Pervaporation oder der Einsatz von Absorptionsmitteln beinhaltet. Besonders bevorzugt im Sinne der vorliegenden Erfindung wird das Lösungs- oder Suspendiermittel ausgewählt aus Alkoholen, Ketonen oder Ethern, besonders bevorzugt aus tertiären Alkoholen, da diese einen besonders hohen Siedepunkt aufweisen. Besonders an dieser Stelle ist 2-Methyl-2-butanol, Dipropylether, 2-Butanon oder Ethylenglykoldimethylether zu nennen.

Das Lösungs- oder Suspendiermittel kann auch Emulgatoren bzw. Tenside, die als zusätzliche Dispergiermittel für die Ascorbinsäure dienen, enthalten. Im Sinne der Erfindung sind tensidische Verbindungen, wie z. B. nichtionische Tenside, Fettsäure- oder Fettalkoholethoxylate, Alkylpolyglucoside oder Polyglycerinester, anionische Tenside wie Fettsäuresalze, Alkylsulfate, Alkylphosphate sowie die Fettsäureascorbylester selbst, und zwitterionische Tenside, wie Cocoamidopropylbetain oder Aminooxide zu nennen.

Im Anschluß an die Reaktion, die vorzugsweise bei einer Temperatur im Bereich von 20 bis 90 °C, insbesondere in einem Temperaturbereich von 40 bis 70 °C durchgeführt wird, kann der erhaltene Ester nach an sich bekannten Verfahren aufbereitet werden. Die Veresterungsreaktion selbst wird vorzugsweise bei einem Druck von weniger als 1 bar, insbesondere von weniger als 400 mbar, vorzugsweise weniger als 250 mbar durchgeführt. Je geringer der Druck ist, desto geringer ist bekanntermaßen die erforderliche Temperatur, um den Reaktionsalkohol destillativ aus dem Gemisch zu entfernen.

Mit Hilfe der vorliegenden Erfindung ist es möglich, hochkonzentrierte Suspensionen von Ascorbinsäure in dem gewählten Lösungs- oder Suspendiermittel umzusetzen. Besonders bevorzugt im Sinne der vorliegenden Erfindung wird daher Ascorbinsäure in einer Menge von 1,5 bis 70 Gew.-%, insbesondere 1,5 bis 50 Gew.-%, bezogen auf die Menge des Lösungs- oder Suspendiermittels eingesetzt.

### Ausführungsbeispiele:

### Beispiel 1:

### 6-Ascorbylpalmitat

5,76 g Ascorbinsäure wurde in 44,23 g Methylpalmitat und 144 ml 2-Methyl-2-Butanol bei 70 °C gerührt. Dann wurde 1 g des Katalysators Novozym®435 zugegeben und ein Vakuum von 200 mbar angelegt. Während der 15stündigen Reaktionsdauer wurde das entstehende Methanol über eine Vigreux-Kolonne abdestilliert. Nach Abschluß der Reaktion wurde der Katalysator abfiltiert und das 2-Methyl-2-Butanol abdestilliert. Man gab zu dem Rückstand Hexan, wodurch das unlösliche Produkt 6-Ascorbylpalmitat ausgefällt wurde. Man erhielt 10,8 g des Produkts, entsprechend einer Ausbeute von 80 %, bezogen auf Ascorbinsäure.

### Beispiel 2:

### 6-Ascorbylricinolat

5,5 g Ascorbinsäure wurde in 39 g Methylricinolat und 155,5 ml 2-Methyl-2-Butanol bei 70 °C gerührt. Dann wurde 1 g des Katalysators Novozym®435 zugegeben und ein Vakuum von 250 mbar angelegt. Während der 20-stündigen Reaktionsdauer wurde das entstehende Methanol über eine Vigreux-Kolonne abgestilliert. Danach wurde der Katalysator abfiltiert und das 2-Methyl-2-Butanol abdestilliert. Das Produkt wurde mit Hexan gereinigt. Man erhielt 11,8 g des Produkts, entsprechend einer Ausbeute von 83 %, bezogen auf Ascorbinsäure.

## Patentansprüche

1. Verfahren zur enzymatischen Herstellung von regioselektiven (6-Ascorbyl) Fettsäureestern der Ascorbinsäure, wobei man Ascorbinsäure und Fettsäurealkylester in einem Lösungs- oder Suspendiermittel bei gegenüber der Atmosphäre vermindertem Druck und Temperaturen oberhalb des Siedepunktes des entstehenden Reaktionsalkohols in Gegenwart von Hydrolasen die ausgewählt sind aus Lipasen, Esterasen oder Proteasen umsetzt.

2. Verfahren nach Anspruch 1, **dadurch gekennzeichnet, daß** man Ascorbinsäure mit Fettsäurealkylestern umsetzt, die ausgewählt sind aus geradkettigen oder verzweigten, einfach oder mehrfach ungesättigten, Hydroxysubstituierten oder unsubstituierten Fettsäureresten mit 8 bis 24 C-Atomen.

3. Verfahren nach Anspruch 1, **dadurch gekennzeichnet, daß** man Fettsäurealkylester einsetzt, deren Fettsäurereste 12 bis 18 C-Atome aufweisen.

4. Verfahren nach Anspruch 1, **dadurch gekennzeichnet, daß** man Fettsäurealkylester einsetzt, deren Alkylreste 1 bis 3 C-Atome aufweisen.

5. Verfahren nach Anspruch 1, **dadurch gekennzeichnet, daß** man ungesättigte, hydroxy-substituierte Fattsäurealkylester einsetzt.

6. Verfahren nach Anspruch 5, **dadurch gekennzeichnet, daß** man Ricinolsäureester von Ascorbinsäure herstellt.

7. Verfahren nach einem der Ansprüche 1 bis 6, **dadurch gekennzeichnet, daß** man das Stoffmengenverhältnis von Ascorbinsäure zu Fettsäurealkylester im Bereich von 10 mol zu 1 mol bis 1 mol zu 10 mol einstellt.

8. Verfahren nach Anspruch 7, **dadurch gekennzeichnet, daß** man das Stoffmengenverhältnis im Bereich von 1 mol zu 1 mol bis 1 mol zu 5 mol einstellt.

9. Verfahren nach einem der Ansprüche 1 bis 8, **dadurch gekennzeichnet, daß** man ein Lösungs- oder Suspendiermittel einsetzt, dessen Siedepunkt oberhalb, insbesondere wenigstens 10 °C oberhalb des Siedepunkts des entstehenden des Reaktionsalkohols liegt.

10. Verfahren nach einem der Ansprüche 1 bis 9, **dadurch gekennzeichnet, daß** man ein Lösungs- oder Suspendiermittel auswählt aus Ketonen und Ethern, insbesondere tertiären Alkoholen, vorzugsweise 2-Methyl-2-butanol, Dipropylether, 2-Butanon oder Ethylenglykoldimethylether.

11. Verfahren nach einem der Ansprüche 1 bis 10, **dadurch gekennzeichnet, daß** man die Reaktion bei einer Temperatur im Bereich von 20 bis 90 °C durchführt.

12. Verfahren nach einem der Ansprüche 1 bis 11, **dadurch gekennzeichnet, daß** man die Reaktion bei einem Druck von weniger als 1 bar durchführt.

13. Verfahren nach einem der Ansprüche 1 bis 12, **dadurch gekennzeichnet, daß** man Ascorbinsäure in einer Menge von 1,5 bis 70 Gew.-%, bezogen auf die Menge des Lösungs- oder Suspendiermittels, einsetzt.

## Claims

1. Process for the enzymatic preparation of regioselective (6-ascorbyl) fatty acid esters of ascorbic acid, in which ascorbic acid and fatty acid alkyl esters are reacted in a solvent or suspending agent at reduced pressure in relation to that of the atmosphere and temperatures above the boiling point of the resulting alcohol of reaction in the presence of hydrolases selected from lipases, esterases or proteases.

2. Process according to Claim 1, **characterized in that** ascorbic acid is reacted with fatty acid alkyl esters which are selected from straight-chain or branched, mono- or polyunsaturated, hydroxy-substituted or unsubstituted fatty acid radicals having 8 to 24 C atoms.

3. Process according to Claim 1, **characterized in that** fatty acid alkyl esters are employed whose fatty acid radicals have 12 to 18 C atoms.

4. Process according to Claim 1, **characterized in that** fatty acid alkyl esters are employed whose alkyl radicals have 1 to 3 C atoms.

5. Process according to Claim 1, **characterized in that** unsaturated, hydroxy-substituted fatty acid alkyl esters are employed.

6. Process according to Claim 5, **characterized in that** ricinoleic acid esters of ascorbic acid are prepared.

7. Process according to one of Claims 1 to 6, **characterized in that** the quantitative ratio of ascorbic acid to fatty acid alkyl ester is set in the range from 10 mol:1 mol to 1 mol:10 mol.

8. Process according to Claim 7, **characterized in that** the quantitative ratio is set in the range from 1 mol-1 mol to 1 mol-5 mol.

9. Process according to one of Claims 1 to 8, **characterized in that** a solvent or suspending agent is employed whose boiling point is above, in particular at least 10°C above, the boiling point of the resulting alcohol of reaction.

10. Process according to one of Claims 1 to 9, **characterized in that** a solvent or suspending agent is selected from ketones and ethers, in particular tertiary alcohols, preferably 2-methyl-2-butanol, dipropyl ether, 2-butanone and ethylene glycol dimethyl ether.

11. Process according to one of Claims 1 to 10, **characterized in that** the reaction is carried out at a temperature in the range from 20 to 90°C.

12. Process according to one of Claims 1 to 11, **characterized in that** the reaction is carried out at a pressure of less than 1 bar.

13. Process according to one of Claims 1 to 12, **characterized in that** ascorbic acid is employed in an amount from 1.5 to 70% by weight, based on the amount of the solvent or suspending agent.

## Revendications

1. Procédé pour la production enzymatique d'esters d'acide gras de l'acide ascorbique régio-sélectifs (6-ascorbyle), dans lequel on transforme de l'acide ascorbique et des esters alkyliques d'acides gras dans un solvant ou un agent de mise en suspension à une pression réduite par rapport à la pression atmosphérique et à des températures supérieures au point de fusion de l'alcool de réaction formé en présence d'hydrolases qui sont choisies parmi les lipases, les estérases ou les protéases.

2. Procédé selon la revendication 1, **caractérisé en ce qu'**on transforme de l'acide ascorbique avec des esters alkyliques d'acides gras qui sont choisis parmi les radicaux d'acides gras linéaires ou ramifiés, monoinsaturés ou polyinsaturés, substitués par hydroxy ou non substitués, comprenant 8 à 24 atomes de carbone.

3. Procédé selon la revendication 1, **caractérisé en ce qu'**on utilise des esters alkyliques d'acides gras dont les radicaux d'acides gras présentent 12 à 18 atomes de carbone.

4. Procédé selon la revendication 1, **caractérisé en ce qu'**on utilise des esters alkyliques d'acides gras dont les radicaux alkyle présentent 1 à 3 atomes de carbone.

5. Procédé selon la revendication 1, **caractérisé en ce qu'**on utilise des esters alkyliques d'acides gras insaturés, substitués par hydroxy.

6. Procédé selon la revendication 5, **caractérisé en ce qu'**on produit l'ester d'acides d'huile de ricin d'acide ascorbique.

7. Procédé selon l'une quelconque des revendications 1 à 6, **caractérisé en ce qu'**on règle un rapport de flux de quantités d'acide ascorbique à ester alkylique d'acide gras dans la plage de 10 moles:1 mole à 1 mole:10 moles.

8. Procédé selon la revendication 7, **caractérisé en ce qu'**on règle un rapport de quantités dans la plage de 1 mole:1 mole à 1 mole:5 moles.

9. Procédé selon l'une quelconque des revendications 1 à 8, **caractérisé en ce qu'**on utilise un solvant ou un agent de mise en suspension dont le point de fusion est supérieur, en particulier d'au moins 10°C, au point de fusion de l'alcool de réaction formé.

10. Procédé selon l'une quelconque des revendications 1 à 9, **caractérisé en ce qu'**on choisit un solvant ou un agent de mise en suspension parmi les cétones et les éthers, en particulier les alcools tertiaires, de préférence le 2-méthyl-2-butanol, le dipropyléther, la 2-butanone ou l'éthylèneglycoldiméthyléther.

11. Procédé selon l'une quelconque des revendications 1 à 10, **caractérisé en ce qu'**on réalise la réaction à une température dans la plage de 20 à 90°C.

12. Procédé selon l'une quelconque des revendications 1 à 11, **caractérisé en ce qu'**on réalise la réaction à une pression inférieure à 1 bar.

13. Procédé selon l'une quelconque des revendications 1 à 12, **caractérisé en ce qu'**on utilise l'acide ascorbique en une quantité de 1,5 à 70% en poids par rapport à la quantité de solvant ou d'agent de mise en suspension.
